# EUROPEAN PATENT APPLICATION

(11) **EP 1 803 809 A2**
(43) Date of publication of application: **04.07.2007**
(21) Application number: 06291283.7
(22) Date of filing: 08.08.2006
(51) Int. Cl.: C12N 9/02, C07K 16/40

(54) **Modulation of peroxisome proliferator-activated receptors**

(30) Priority: 12.08.2005 US 707897 P
(71) Applicant: Abgenomics Corporation, Taipei, Taiwan 114 (TW)
(72) Inventor: Lin, Rong Wha, Da-an District, Taipei, Taiwan 106 (CN); Chuang, Lee-Ming, Xindian City, Taipei Country, Taiwan 231 (CN)
(74) Representative: Nargolwalla, Cyra

(57) **Abstract**

A method of treating a peroxisome proliferators-activated receptors related disease by administering to a subject in need thereof an effective amount of a modulator of 15-keto prostaglandin-Δ¹³-reductase. Also disclosed are methods of identifying a compound for inhibiting activity of the reductase and of lowering blood glucose levels by administering to a subject an effective amount of a reductase inhibitor.

## Description

### CROSS REFERENCE TO RELATED APPLICATION

Pursuant to 35 U.S.C. § 119(e), this application claims priority to U.S. Provisional Application Serial No. 60/707,897, filed August 12, 2005, the contents of which are hereby incorporated by reference.

### BACKGROUND

Peroxisome proliferator-activated receptors (PPARs) belong to a family of nuclear receptors that regulate lipid and glucose metabolism. Three mammalian PPARs have been identified, i.e., PPAR-alpha, PPAR-gamma, and PPAR-delta. Upon activation by either dietary fatty acids or their metabolic derivatives, PPARs trigger a cascade of transcriptional events leading to altered lipid and glucose metabolism. For example, upon activation, PPAR-gamma, highly expressed in adipose tissues, promotes glucose uptake and lowers blood glucose levels.

Given their roles in lipid and glucose metabolism, PPARs are promising therapeutic targets of diseases, e.g., type II diabetes, obesity, dyslipidemia, coronary heart disease, inflammatory disease, and cancer. Synthetic PPAR-gamma agonists, i.e., AVANDIA and ACTOS, have been used to treat type II diabetes. Another synthetic PPAR-alpha agonist, i.e., Fibrate, has been used to treat dyslipidemia. See Lehmann, et al., J Biol Chem, (1995) 270:12953-12956; Fruchart, et al., Curr. Opin. Lipdol. (1999) 10:245-257. However, most PPAR therapeutics have limited efficacy and significant side effects.

There is a need to develop more effective drugs for controlling lipid and glucose metabolism via modulation of PPAR activity.

### SUMMARY

This invention relates to methods of treating PPAR related diseases via modulation of PPAR activity in a subject.

In one aspect, this invention features an isolated polypeptide, PGR3/ZADH2, that reduces 15-keto prostaglandin but not leukotriene B4. Prostaglandin (PG) is a class of physiological mediators characterized by a central ring and side chains of varying degrees of unsaturation. Examples of 15-keto prostaglandin include but are not limited to 15-keto PGE₂, 15-keto PGE₁, 15-keto PGF_{2α}, and 15-keto PGF_{1α}.

In another aspect, this invention features an antibody that binds specifically to the polypeptide described above. The antibody, either polyclonal or monoclonal, may bind to a fragment of the polypeptide.

In still another aspect, this invention features a double-stranded ribonucleic acid (dsRNA), as well as a DNA vector encoding it, for inhibiting expression of a polypeptide with 15-keto prostaglandin-Δ¹³-reductase activity. 15-keto prostaglandin-Δ¹³-reductase refers to an enzyme that catalyzes the conversion of a 15-keto prostaglandin to 13,14-dihydro-15-keto prostaglandin by reducing the Δ¹³ double bond of the prostaglandin. Examples of 15-keto prostaglandin-Δ¹³-reductases include 15-keto prostaglandin-Δ¹³-reductase/leukotriene B4 12-hydroxydehydrogenase (PGR/LTB4DH), zinc binding alcohol dehydrogenase 1 (PGR2/ZADH1), and zinc binding alcohol dehydrogenase 2 (PGR3/ZADH2). The dsRNA contains two strands of polyribonucleotide. The first strand contains a sequence that is identical to 19 to 49 consecutive nucleotides of a nucleic acid that encodes 15-keto prostaglandin-Δ¹³-reductase. The second strand is complementary to the first strand. Preferably, at least one end of the dsRNA has an overhang of 1 to 4 nucleotides. In one example, the first strand contains SEQ ID NO: 9 or 10, or its fragment. The 15-keto prostaglandin-Δ¹³-reductase can be PGR/LTB4DH, PGR2/ZADH1, or PGR3/ZADH2.

In still another aspect, this invention features a method of treating a PPAR related disease such as type II diabetes, obesity, dyslipidemia, coronary heart disease, inflammatory disease, and cancer. The method includes administering to a subject an effective amount of a PGR3/ZADH2 modulator. A PGR3/ZADH2 modulator refers to a molecule or a complex of molecules that affects activity or expression of PGR3/ZADH2. A modulator can be a 15-keto prostaglandin. It can also be an inhibitor that suppresses either activity or expression of PGR3/ZADH2, such as a large molecule inhibitor (e.g., the above-described dsRNA) or a small molecule inhibitor (e.g., tetrahydroxyflavone, trihydroxyflavone, 2-hydroxy-trimethoxychalcone, 2-hydroxy-benzyl cinnamate, or benzyl hydroxylcinnamate). Examples of small molecule inhibitors include compounds 1-49 described below.

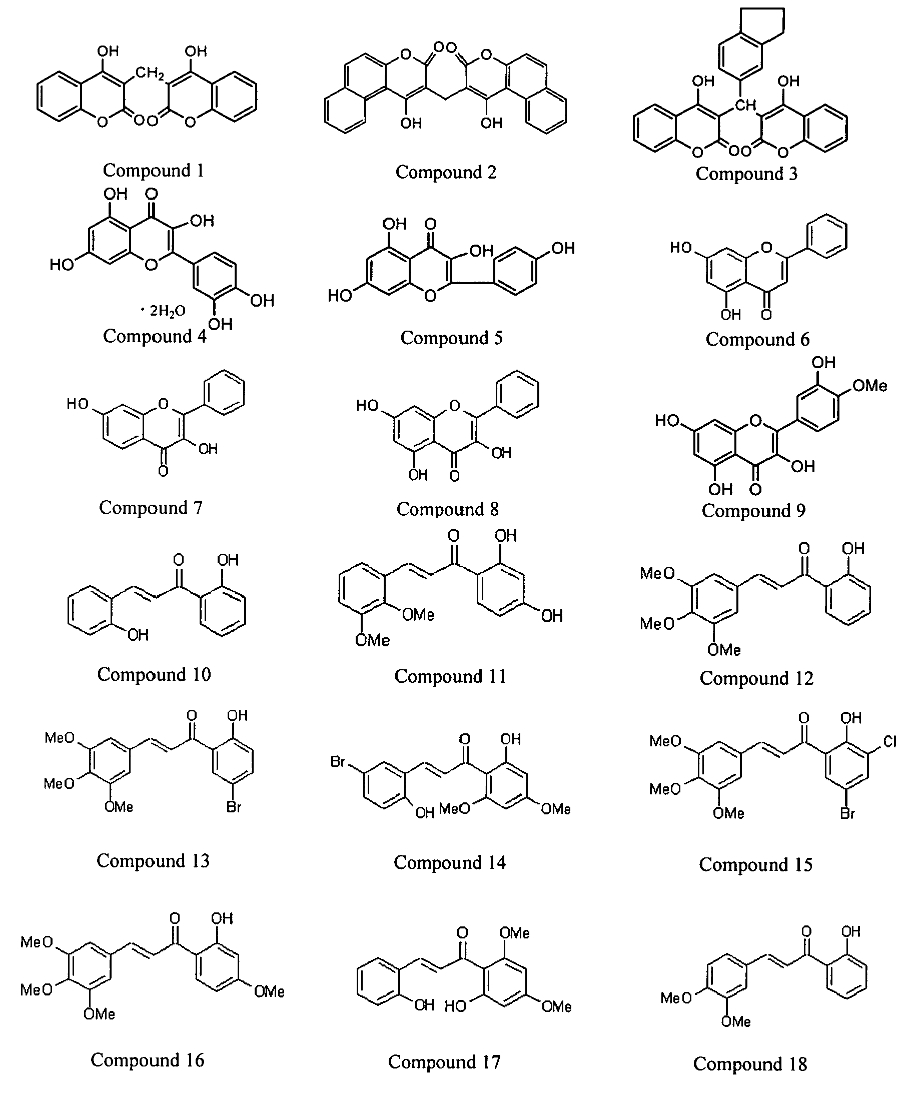

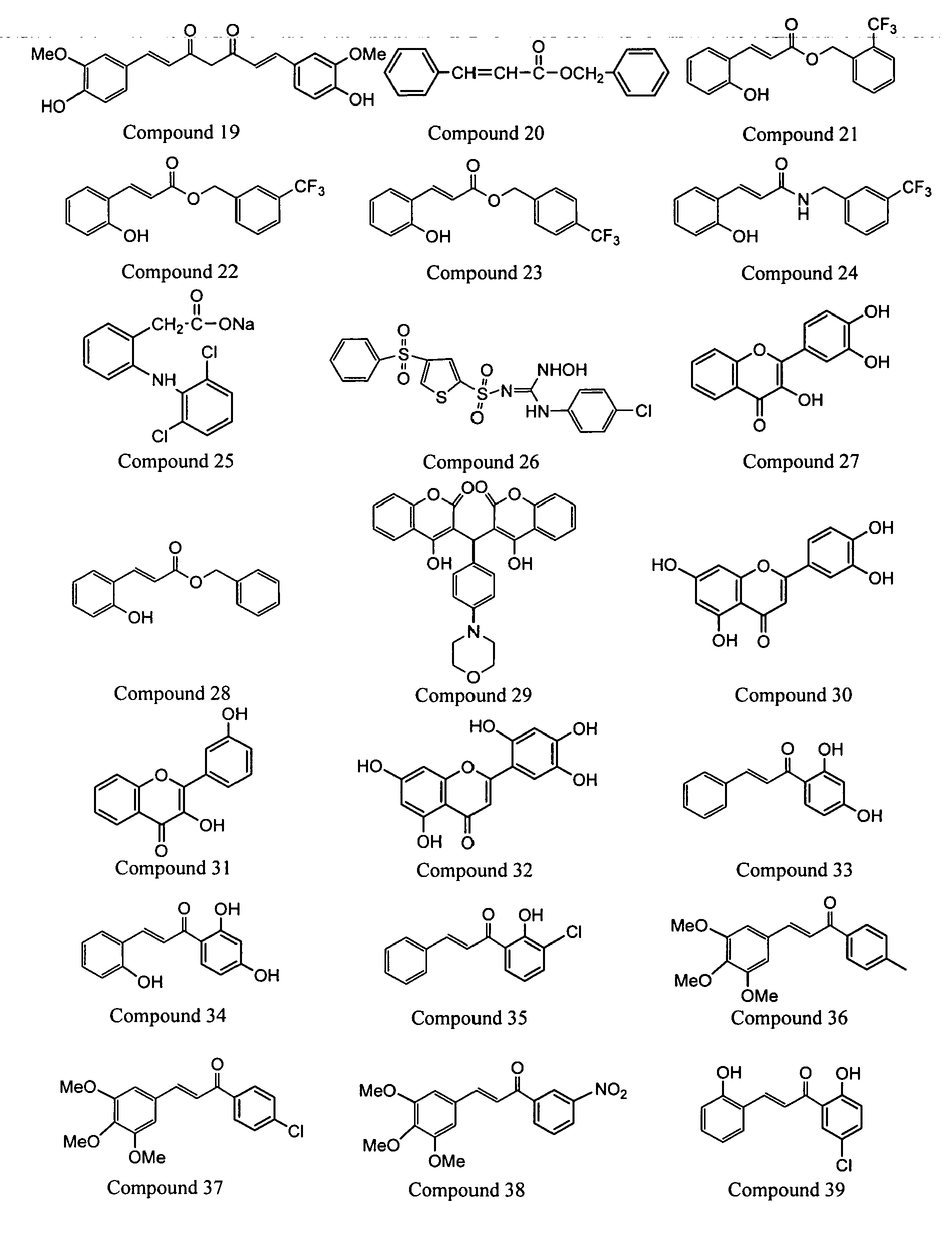

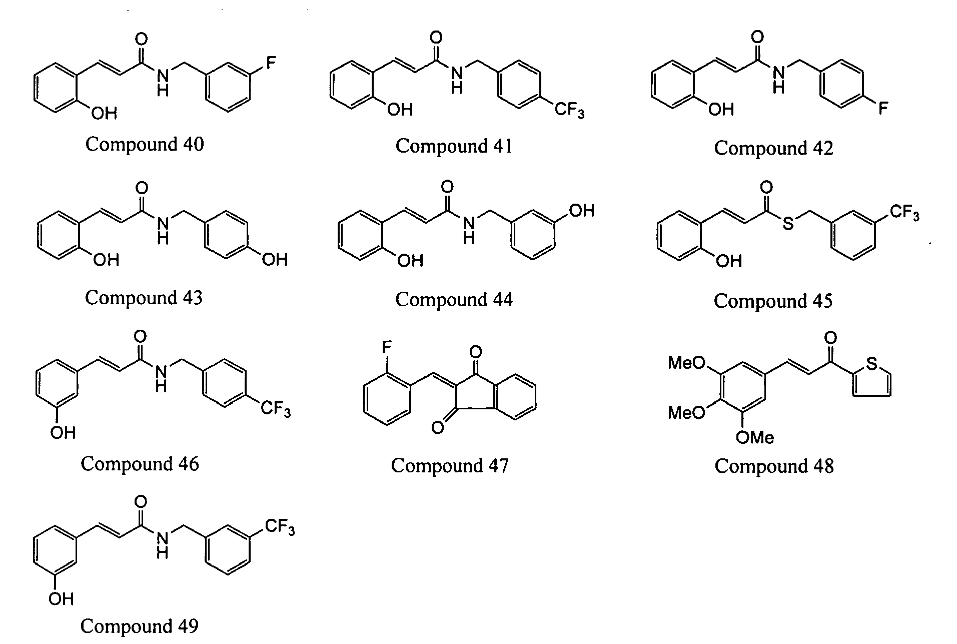

In still another aspect, this invention features a method of treating a PPAR-related disease. The method includes administering to a subject in need thereof an effective amount of a compound of formula (I): 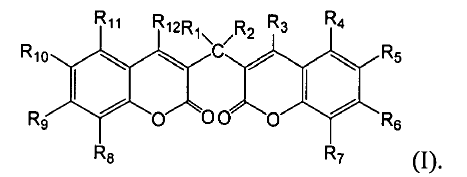 In formula (I), each of R₁ and R₂, independently, is H, halo, ORₐ, C₁-C₁₀ alkyl, C₃-C₂₀ cycloalkyl, C₃-C₂₀ heterocycloalkyl, aryl, or heteroaryl; and each of R₃, R₄, R₅, R₆, R₇, R₈, R₉, R₁₀, R₁₁, and R₁₂, independently, is H, halo, OR_{b}, C₁-C₁₀ alkyl, C₃-C₂₀ cycloalkyl, C₃-C₂₀ heterocycloalkyl, aryl, or heteroaryl; or R₄ and R₅, taken together, are C₃-C₂₀ cycloalkyl, C₃-C₂₀ heterocycloalkyl, aryl, or heteroaryl; or R₁₀ and R₁₁, taken together, are C₃-C₂₀ cycloalkyl, C₃-C₂₀ heterocycloalkyl, aryl, or heteroaryl; in which each of Rₐ and R_{b}, independently, is H, C₁-C₁₀ alkyl, C₃-C₂₀ cycloalkyl, C₃-C₂₀ heterocycloalkyl, aryl, or heteroaryl.

In a subset of compounds of formula (I), each of R₁ and R₂, independently, is H, phenyl fused with cyclopentyl, or phenyl substituted with heteroaryl. In another subset of compounds of formula (I), R₃ and R₁₂ is OH.

The term "alkyl" refers to a saturated or unsaturated, linear or branched hydrocarbon moiety, such as -CH₃, -CH₂-CH=CH₂, or branched -C₃H₇. The term "cycloalkyl" refers to a saturated or unsaturated, non-aromatic, cyclic hydrocarbon moiety, such as cyclohexyl or cyclohexen-3-yl. The term "heterocycloalkyl" refers to a saturated or unsaturated, non-aromatic, cyclic moiety having at least one ring heteroatom (e.g., N, O, or S), such as 4-tetrahydropyranyl or 4-pyranyl. The term "aryl" refers to a hydrocarbon moiety having one or more aromatic rings. Examples of aryl moieties include phenyl (Ph), phenylene, naphthyl, naphthylene, pyrenyl, anthryl, and phenanthryl. The term "heteroaryl" refers to a moiety having one or more aromatic rings that contain at least one heteroatom (e.g., N, O, or S). Examples of heteroaryl moieties include furyl, furylene, fluorenyl, pyrrolyl, thienyl, oxazolyl, imidazolyl, thiazolyl, pyridyl, pyrimidinyl, quinazolinyl, quinolyl, isoquinolyl and indolyl.

Alkyl, alkylene, heteroalkylene, cycloalkyl, heterocycloalkyl, aryl, and heteroaryl mentioned herein include both substituted and unsubstituted moieties, unless specified otherwise. Possible substituents on cycloalkyl, heterocycloalkyl, aryl, and heteroaryl include, but are not limited to, C₁-C₁₀ alkyl, C₂-C₁₀ alkenyl, C₂-C₁₀ alkynyl, C₃-C₈ cycloalkyl, C₅-C₈ cycloalkenyl, C₁-C₁₀ alkoxy, aryl, aryloxy, heteroaryl, heteroaryloxy, amino, C₁-C₁₀ alkylamino, C₁-C₂₀ dialkylamino, arylamino, diarylamino, hydroxyl, halogen, thio, C₁-C₁₀ alkylthio, arylthio, C₁-C₁₀ alkylsulfonyl, arylsulfonyl, acylamino, aminoacyl, aminothioacyl, amidino, guanidine, ureido, cyano, nitro, acyl, thioacyl, acyloxy, carboxyl, and carboxylic ester. On the other hand, possible substituents on alkyl, alkylene, or heteroalkylene include all of the above-recited substituents except C₁-C₁₀ alkyl, C₂-C₁₀ alkenyl, and C₂-C₁₀ alkynyl. Cycloalkyl, heterocycloalkyl, aryl, and heteroaryl can also be fused with each other.

In still another aspect, this invention features a method of treating a PPAR-related disease by administering to a subject in need thereof an effective amount of a compound of formula (II): 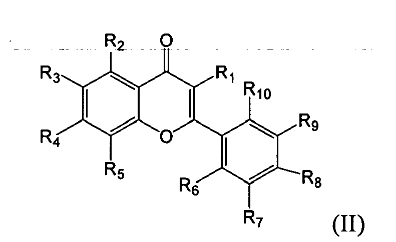 In formula (II), each of R₁, R₂, R₃, R₄, R₅, R₆, R₇, R₈, R₉, and R₁₀, independently, is H, OR, C₁-C₁₀ alkyl, C₃-C₂₀ cycloalkyl, C₃-C₂₀ heterocycloalkyl, aryl, or heteroaryl; in which R is H, C₁-C₁₀ alkyl, C₃-C₂₀ cycloalkyl, C₃-C₂₀ heterocycloalkyl, aryl, or heteroaryl. In a subset of compounds of formula (II), each of R₁, R₂, R₃, R₄, R₅, R₆, R₇, R₈, R₉, and R₁₀, independently, is H or OH.

In still another aspect, this invention features a method of treating a PPAR-related disease by administering to a subject in need thereof an effective amount of a compound of formula (III): 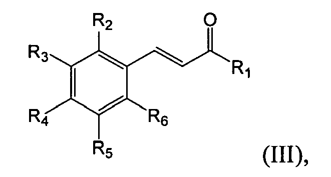 In formula (III), R₁ is C₁-C₁₀ alkyl, C₃-C₂₀ cycloalkyl, C₃-C₂₀ heterocycloalkyl, aryl, heteroaryl, ORₐ, NRₐR_{b}, or SRₐ; and each of R₂, R₃, R₄, R₅, and R₆, independently, is H, halo, OR, NR_{c}R_{d}, C₁-C₁₀ alkyl, C₃-C₂₀ cycloalkyl, C₃-C₂₀ heterocycloalkyl, aryl, or heteroaryl; in which each of Rₐ, R_{b}, R_{c}, and R_{d}, independently, is H, C₁-C₁₀ alkyl, C₃-C₂₀ cycloalkyl, C₃-C₂₀ heterocycloalkyl, aryl, or heteroaryl. In a subset of compounds of formula (III), R₁ can be phenyl substituted with halo, OR, NO₂, or C₁-C₁₀ alkyl, in which R is H or C₁-C₁₀ alkyl. In another subset of compounds of formula (III), R₁ can be ORₐ, NRₐR_{b}, or SRₐ, in which Rₐ is C₁-C₁₀ alkyl substituted with phenyl, phenyl being optionally substituted with CF₃, F, or OH; and R_{b} is H. In still another subset of compounds of formula (III), R₁ is heteroaryl, or C₁-C₁₀ alkyl substituted with C(O)R, in which R is H or C₁-C₁₀ alkyl.

Also within the scope of this invention is a method of lowering blood glucose levels in a subject. The method includes administering to the subject an effective amount of one of the above-described PGR3/ZADH2 inhibitors. The inhibitor can suppress either activity or expression of PGR3/ZADH2.

This invention further features a method of identifying a compound that inhibits PGR3/ZADH2 activity. Inhibition refers to suppression of either activity or expression of PGR3/ZADH2. The method includes providing a system containing PGR3/ZADH2, contacting a compound with the system, determining the PGR3/ZADH2 activity, and comparing the activity with that obtained in the same manner except that the compound is absent. In one embodiment, the system is a cell containing a gene that expresses PGR3/ZADH2 and the activity is determined by measuring expression activity of the gene. In another embodiment, the system is a cell-free solution that contains PGR3/ZADH2 and the activity is determined by measuring enzymatic PGR3/ZADH2 activity of the cell-free solution.

The details of one or more embodiments of the invention are set forth in the description below. Other features, objects, and advantages of the invention will be apparent from the description and from the claims.

### DETAILED DESCRIPTION

The present invention is based on the discovery of 15-keto prostaglandin-Δ¹³-reductase 3 (PGR3/ ZADH2), a member of the 15-keto prostaglandin-Δ¹³-reductase family. It was found unexpectedly that PPAR activity can be controlled by its substrates and inhibitors. These substrates and inhibitors are useful for treating PPAR related diseases, e.g., type II diabetes, obesity, dyslipidemia, coronary heart disease, inflammatory disease, and cancer.

Contemplated within the scope of this invention is an isolated human polypeptide of SEQ ID NO:1 or its functional equivalent that reduces 15-keto prostaglandin. Shown below is its amino acid sequence (SEQ ID NO: 1), as well as the encoding nucleotide sequence (i.e., SEQ ID NO:2).

1   M  L  R  L  V  P  T  G  A  R  A  I  V  D  M  S  Y  A  R  H
1  ATGCTGCGGCTGGTGCCCACCGGGGCCCGGGCCATCGTGGACATGTCGTACGCCCGCCAC

21 F L D F Q G S A I P Q A M Q K L V V T R
61 TTCCTGGACTTCCAGGCTCCGCCATTCCCCAAGCCATGCAGAAGCTGGTGGTGACCCGG
41 L S P N F R E A V T L S R D C P V P L P
121 CTGAGCCCCAACTTCCGCGAGGCCGTCACCCTGAGCCGGGACTGCCCGGTGCCGCTCCCC
61 G D G D L L V R N R F V G V N A S D I N
181 GGGGACGGAGACCTCCTCGTCCGGAACCGATTTGTTGGTGTTAACGCATCTGACATCAAC
81 Y S A G R Y D P S V K P P F D I G F E G
241 TATTCAGCAGGCCGCTATGACCCCTCAGTTAAGCCTCCCTTTGACATAGGTTTCGAAGGC
101 I G E V V A L G L S A S A R Y T V G Q A
301 ATTGGGGAGGTGGTGGCCCTAGGCCTCTCTGCTAGTGCCAGATACACAGTTGGCCAAGCT
121 V A Y M A P G S F A E Y T V V P A S I A
361 GTGGCTTACATGGCACCTGGTTCTTTTGCTGAGTACACAGTTGTGCCTGCCAGCATTGCA
141 T P V P S V K P E Y L T L L V S G T T A
421 ACTCCAGTGCCCTCAGTGAAACCCGAGTATCTTACCCTGCTGGTAAGTGGCACCACCGCA
161 Y I S L K E L G G L S E G K K V L V T A
481 TACATCAGCCTGAAAGAGCTCGGAGGACTGTCGGAAGGAAAAAAGTTTTGGTGACAGCA
181 A A G G T G Q F A M Q L S K K A K C H V
541 GCAGCTGGGGGAACGGGCCAGTTTGCCATGCAGCTTTCAAAGAAGGCAAAGTGCCATGTA
201 I G T C S S D E K S A F L K S L G C D R
601 ATTGGAACCTGCTCTTCTGATGAAAAGTCTGCTTTTCTGAAATCTCTTGGCTGTGATCGT
221 P I N Y K T E P V G T V L K Q E Y P E G
661 CCTATCAACTATAAAACTGAACCCGTAGGTACCGTCCTTAAGCAGGAGTACCCTGAAGGT
241 V D V V Y E S V G G A M F D L A V D A L
721 GTCGATGTGGTCTATGAATCTGTTGGGGGAGCCATGTTTGACTTGGCTGTAGACGCCCTG
261 A T K G R L I V I G F I S G Y Q T P T G
781 GCTACGAAAGGGCGCTTGATAGTAATAGGGTTTATCTCTGGCTACCAAACTCCTACTGGC
281 L S P V K A G T L P A K L L K K S A S V
841 CTTTCGCCTGTGAAAGCAGGAACATTGCCAGCCAAACTGCTCAAGAAATCTGCCAGCGTA
301 Q G F F L N H Y L S K Y Q A A M S H L L
901 CAGGGCTTCTTCCTGAACCATTACCTTTCTAAGTATCAAGCAGCCATGAGCCACTTGCTC
321 E M C V S G D L V C E V D L G D L S P E
961 GAGATGTGTGTGAGCGGAGACCTGGTTTGTGAGGTGGACCTTGGAGATCTGTCTCCAGAG
341 G R F T G L E S I F R A V N Y M Y M G K
1021 GGCAGGTTTACTGGCCTGGAGTCCATATTCCGTGCTGTCAATTATATGTACATGGGAAAA
361 N T G K I V V E L P H S V N S K L SEQ ID NO:1
1081 AACACTGGAAAAATTGTAGTTGAATTACCTCACTCTGTCAACAGTAAGCTGTAA SEQ ID NO:2

Also within the scope of this invention is an isolated mouse polypeptide or its functional equivalent that reduces 15-keto prostaglandin. Shown below is the amino acid sequence of the mouse polypeptide (SEQ ID NO:3) as well as the encoding nucleotide sequence (i.e. SEQ ID NO:4).

SEQ ID NO:3

MLRLAAAGARAIVDMSYARHFLDFQGSAIPRTMQKLVVTRLSPNFHEAVTLRRDCPVPLPGDGDLLVRNRFV
GINASDINYSAGRYDPSLKPPFDIGFEGIGEVVALGLSASARYTVGQAVAYMAPGSFAEYTVVPASIAIPMP
SVKPEYLTMLVSGTTAYLSLEELGELSEGKKVLVTAAAGGTGQFAVQLSKIAKCHVIGTCSSDEKAAFLKSI
GCDRPINYRTEPVETVLKQEYPEGVDVVYESVGGAMFDLAVDALATKGRLIVIGFISGYQSPTGLSPIKAGV
LPTKLLKKSASLRGFFLNHYFSKYQAAMERLLELYARGDLVCEVDLGHLAPDGRFIGLESVFQAVDYMYTGK
NTGKLVVELPHPVSSKL

SEQ ID NO:4

at gctgaggctg gcggccgccg gggcccgagc catcgtggac atgtcgtacg
53 cccgtcactt cctggacttc cagggctccg ccatcccccg aaccatgcag aagctggtgg
113 tgacccggct gagccctaac ttccacgagg ccgtcaccct gcgccgggac tgcccagtgc
173 cgctccccgg ggacggagac ctcctcgtcc ggaaccgatt cgttggtatt aacgcatctg
233 acatcaacta ttcggctggc cgctacgacc cgtccctgaa gccacccttt gacataggtt
293 ttgaagggat tggtgaggtg gtggccttag gcctctctgc tagtgctagg tacacagtgg
353 gccaggctgt ggcttatatg gctcctggtt cctttgctga gtatacagtg gtgcctgcta
413 gcattgcaat tcccatgcct tcagtgaaac cagagtatct caccatgctg gttagtggca
473 ccactgcata cctcagcctg gaagagcttg gggaactgtc agaagggaag aaagttctgg
533 tcacagcagc cgctgggggc acaggccagt ttgctgtgca gctttccaag atagccaagt
593 gccacgttat cggaacctgc tcctcagacg aaaaggcagc ttttctgaaa tcaattgggt
653 gtgatcggcc catcaactac agaacagagc ctgtggagac ggttctgaag caggagtacc
713 ctgaaggcgt tgacgtagtc tatgagtctg ttgggggagc catgtttgac ctggctgtgg
773 atgccttggc caccaaaggg cgcttgatag tgattgggtt tatctctggc taccaaagcc
833 ctacaggact ctcaccaata aaagcgggag ttttgccaac caagctcctg aagaagtcgg
893 ccagcctcag gggtttcttt ctgaaccact acttctccaa gtaccaggct gccatggaac
953 gtttgctgga gctgtacgct cgtggggacc tggtgtgtga ggtggacctg ggacacctgg
1013 ctccggatgg aaggttcatt ggcctggagt ccgtgtttca ggctgtcgac tatatgtaca
1073 cggggaaaaa tactgggaag cttgttgttg agttaccaca ccctgtcagc agtaagctgt
1133 ga

An isolated polypeptide refers to a polypeptide substantially free from naturally associated molecules, i.e., it is at least 75% (i.e., any number between 75% and 100%, inclusive) pure by dry weight. Purity can be measured by any appropriate standard method, for example, by column chromatography, polyacrylamide gel electrophoresis, or HPLC analysis. An isolated polypeptide of the invention can be purified from a natural source, produced by recombinant DNA techniques, or by chemical methods. The term "functional equivalent" refers to a variant of a polypeptide of SEQ ID NO: or SEQ ID NO:3 that possesses the ability to reduce 15-keto prostaglandin, e.g., a protein having one or more point mutations, insertions, deletions, truncations, or a combination thereof. In one embodiment, an isolated polypeptide of the invention or its functional equivalent contains a sequence that is at least 80% (e.g., 85%, 95%, or 100%, or any other number between 80% and 100%, inclusive) identical to SEQ ID NO: 1 or SEQ ID NO:3. It can be a fusion protein.

15-keto prostaglandin-Δ¹³-reductase activity refers to the enzymatic conversion of 15-keto prostaglandin to 13,14-dihydro-15-keto prostaglandin. The specific activity is determined as follows: 10 µg of a protein preparation to be assayed is incubated at 37°C in a reaction buffer containing 0.1M Tris-HCl (pH 7.4), 0.5 mM NADPH, and 0.57 mM 15-keto PGE₂. The reaction is conducted in the dark for 10 minutes at 37°C and terminated by adding 700 µl of a buffer containing 50 mM potassium hydrogen phthalate, pH 3.0, and 1% Tween 20. 200 µl of a color development reagent, which contains 790 µM indonitrotetrazolium chloride, 60 µM phenazene methosulfate, and 1% Tween 20, is used to oxidize any unreacted NADPH. Absorbance at 490 nm is measured using an ELISA plate reader. A standard curve is generated using reaction buffers containing serially diluted amounts of NADPH. A specific activity of at least 90 nmole/min.mg protein indicates that the polypeptide has 15-keto prostaglandin-Δ¹³-reductase activity.

The nucleotide sequence described above, i.e., SEQ ID NO:2 or SEQ ID NO:4, can be used to express the polypeptide of this invention. A nucleotide sequence refers to a DNA molecule (e.g., a cDNA or genomic DNA), an RNA molecule (e.g., an mRNA), or a DNA or RNA analog. A DNA or RNA analog can be synthesized from nucleotide analogs. The nucleic acid molecule can be single-stranded or double-stranded, but preferably double-stranded. For the purpose of protein expression, one can operatively linked the nucleic acid to suitable regulatory sequences to generate an expression vector. A vector refers to a nucleic acid molecule capable of transporting another nucleic acid to which it has been linked. The vector can be capable of autonomous replication or integrate into a host DNA. Examples of the vector include a plasmid, cosmid, or viral vector. The vector may include a nucleotide sequence in a form suitable for expression of the nucleic acid in a host cell. Preferably the vector includes one or more regulatory sequences operatively linked to the nucleic acid sequence to be expressed. A "regulatory sequence" includes promoters, enhancers, and other expression control elements (e.g., polyadenylation signals). Regulatory sequences include those that direct constitutive expression of a nucleotide sequence, as well as tissue-specific regulatory and/or inducible sequences. The design of the expression vector can depend on such factors as the choice of the host cell to be transformed, the level of expression of protein desired, and the like. The expression vector can be introduced into host cells to produce the polypeptide of this invention. Also within the scope of this invention is a host cell that contains the above-described nucleic acid. Examples include *E. coli* cells, Sf9 insect cells (e.g., using baculovirus expression vectors), yeast cells, or mammalian cells. See e.g., Goeddel, (1990) Gene Expression Technology: Methods in Enzymology 185, Academic Press, San Diego, CA. To produce a polypeptide of this invention, one can culture a host cell in a medium under conditions permitting expression of the polypeptide encoded by a nucleic acid of this invention, and purify the polypeptide from the cultured cell or the medium of the cell. Alternatively, the nucleotide sequence can be transcribed and translated in vitro, for example, using T7 promoter regulatory sequences and T7 polymerase.

The above-described polypeptide can be used to generate antibodies in animals. It is understood that the antibodies can also be generated from a fragment of the polypeptide. Methods of making monoclonal and polyclonal antibodies and fragments thereof in animals are known in the art. See, for example, Harlow and Lane, (1988) Antibodies: A Laboratory Manual, Cold Spring Harbor Laboratory, New York. The term "antibody" includes intact molecules as well as fragments thereof, such as Fab, F(ab')₂, Fv, scFv (single chain antibody), and dAb (domain antibody; Ward, et. al. (1989) Nature, 341,544).

In general, a polypeptide of this invention can be coupled to a carrier protein, such as KLH, mixed with an adjuvant, and injected into a host animal. Antibodies produced in that animal can then be purified by peptide affinity chromatography. Commonly employed host animals include rabbits, mice, guinea pigs, and rats. Various adjuvants that can be used to increase the immunological response depend on the host species and include Freund's adjuvant (complete and incomplete), mineral gels such as aluminum hydroxide, surface active substances such as lysolecithin, pluronic polyols, polyanions, peptides, oil emulsions, keyhole limpet hemocyanin, and dinitrophenol. Useful human adjuvants include BCG (bacille Calmette-Guerin) and Corynebacterium parvum.

Polyclonal antibodies, heterogeneous populations of antibody molecules, are present in the sera of the immunized subjects. Monoclonal antibodies, homogeneous populations of antibodies to a polypeptide of this invention, can be prepared using standard hybridoma technology (see, for example, Kohler et al. (1975) Nature 256, 495; Kohler et al. (1976) Eur J Immunol 6, 511; Kohler et al. (1976) Eur J Immunol 6, 292; and Hammerling et al. (1981) Monoclonal Antibodies and T Cell Hybridomas, Elsevier, N.Y.). In particular, monoclonal antibodies can be obtained by any technique that provides for the production of antibody molecules by continuous cell lines in culture such as described in Kohler et al. (1975) Nature 256, 495 and U.S. Patent No. 4,376,110; the human B-cell hybridoma technique (Kosbor et al. (1983) Immunol Today 4, 72; Cole et al. (1983) Proc. Natl. Acad. Sci. USA 80, 2026, and the EBV-hybridoma technique (Cole et al. (1983) Monoclonal Antibodies and Cancer Therapy, Alan R. Liss, Inc., pp. 77-96). Such antibodies can be of any immunoglobulin class including IgG, IgM, IgE, IgA, IgD, and any subclass thereof. The hybridoma producing the monoclonal antibodies of the invention may be cultivated in vitro or in vivo. The ability to produce high titers of monoclonal antibodies in vivo makes it a particularly useful method of production.

In addition, techniques developed for the production of "chimeric antibodies" can be used. See, e.g., Morrison et al. (1984) Proc. Natl. Acad. Sci. USA 81, 6851; Neuberger et al. (1984) Nature 312, 604; and Takeda et al. (1984) Nature 314:452. A chimeric antibody is a molecule in which different portions are derived from different animal species, such as those having a variable region derived from a murine monoclonal antibody and a human immunoglobulin constant region. Alternatively, techniques described for the production of single chain antibodies (U.S. Patent Nos. 4,946,778 and 4,704,692) can be adapted to produce a phage library of single chain Fv antibodies. Single chain antibodies are formed by linking the heavy and light chain fragments of the Fv region via an amino acid bridge. Moreover, antibody fragments can be generated by known techniques. For example, such fragments include, but are not limited to, F(ab')₂ fragments that can be produced by pepsin digestion of an antibody molecule, and Fab fragments that can be generated by reducing the disulfide bridges of F(ab')₂ fragments. Antibodies can also be humanized by methods known in the art. For example, monoclonal antibodies with a desired binding specificity can be commercially humanized (Scotgene, Scotland; and Oxford Molecular, Palo Alto, Calif.). Fully human antibodies, such as those expressed in transgenic animals are also features of the invention (see, e.g., Green et al. (1994) Nature Genetics 7, 13; and U.S. Patent Nos. 5,545,806 and 5,569,825).

Also within the scope of this invention is a double-stranded ribonucleic acid (dsRNA). This dsRNA can be used to inhibit expression of PGR3/ZADH2. Thus, one can treat a PPAR related disease by administering the dsRNA to a subject. The term "dsRNA" refers to a double-stranded ribonucleic acid that silences gene expression via degradation of a targeted RNA sequence, a process known as RNA interference (RNAi). RNAi has been used to silence gene expression in a wide variety of animal models (including C. elegans, zebrafish, and mouse embryos) and in other biological systems (including explanted chick neural cells and mammalian cell culture). See WO99/32619, WO00/44914, WO00/44914, WO00/44895, WO00/63364, and WO01/36646 A1.

The RNA of this invention can be synthesized by techniques well known in the art. See, e.g., Caruthers et al., 1992, Methods in Enzymology 211, 3-19, Wincott et al., 1995, Nucleic Acids Res. 23, 2677-2684, Wincott et al., 1997, Methods Mol. Bio. 74, 59, Brennan et al., 1998, Biotechnol Bioeng., 61, 33-45, and Brennan, U.S. Patent No. 6,001,311. The RNA can also be transcribed from an expression vector and isolated using standard techniques. The RNA or vector of this invention can be delivered to target cells using method also well known in the art. See, e.g., Akhtar et al., 1992, Trends Cell Bio. 2, 139. For example, it can be introduced into cells using liposomes, hydrogels, cyclodextrins, biodegradable nanocapsules, or bioadhesive microspheres. Alternatively, the RNA or vector is locally delivered by direct injection or by use of an infusion pump. Other approaches include use of various transport and carrier systems, e.g., using conjugates and biodegradable polymers.

This invention also features a method of treating PPAR related diseases by modulating PGR3/ZADH2 activity or expression. The term "treating" refers to administering one or more of the above-described PGR3/ZADH2 modulators, i.e., PGR3/ZADH2 substrates and inhibitors, to a subject who has a PPAR related disease, a symptom of such a disease, or a predisposition toward such a disease, with the purpose to confer a therapeutic effect, e.g., to cure, relieve, alter, affect, ameliorate, or prevent the PPAR related disease, the symptom of it, or the predisposition toward it. "An effective amount" refers to the amount that is required to confer a therapeutic effect on a treated subject. A substrate of PGR3/ZADH2 includes 15-keto PGE₂, 15-keto PGE₁, 15-keto PGF_{2α}, 15-keto PGF_{1α}, and structural analogs thereof.

Examples of PPAR related diseases (or disorders or conditions) include, but are not limited to, type II diabetes, hyperglycemia, low glucose tolerance, Syndrome X, insulin resistance, obesity, lipid disorders, dyslipidemia, hypertriglyceridemia, hypercholesterolemia, low HDL levels, high LDL levels, atherosclerosis (and its sequelae such as angina, claudication, heart attack, or stroke), vascular restenosis, irritable bowel syndrome, inflammatory diseases (e.g., inflammatory bowel disease, rheumatoid arthritis, Crohn's disease, ulcerative colitis, osteoarthritis, multiple sclerosis, asthma, vasculitis, ischemia/reperfusion injury, frostbite, or adult respiratory distress syndrome), pancreatitis, neurodegenerative disease, retinopathy, neoplastic conditions, cancers (e.g., prostate, gastric, breast, bladder, lung, or colon cancer, or adipose cell cancer such as liposarcoma), angiogenesis, Alzheimer's disease, skin disorders (e.g., acne, psoriasis, dermatitis, eczema, or keratosis), high blood pressure, ovarian hyperandrogenism, osteoporosis, and osteopenia.

To treat a PPAR related disease, a pharmaceutical composition containing a PGR3/ZADH2 modulator and a pharmaceutically acceptable carrier can be administered to a subject in need thereof. It can be administered orally or by intravenous infusion, or injected or implanted subcutaneously, intramuscularly, intrathecally, intraperitoneally, intrarectally, intravaginally, intranasally, intragastrically, intratracheally, or intrapulmonarily.

The pharmaceutical composition can be a solution or suspension in a non-toxic acceptable diluent or solvent, such as a solution in 1,3-butanediol. Among the acceptable vehicles and solvents that can be employed are mannitol, water, Ringer's solution, and isotonic sodium chloride solution. In addition, fixed oils are conventionally employed as a solvent or suspending medium (e.g., synthetic mono- or diglycerides). Fatty acid, such as oleic acid and its glyceride derivatives are useful in the preparation of injectables, as are natural pharmaceutically acceptable oils, such as olive oil or castor oil, especially in their polyoxyethylated versions. These oil solutions or suspensions can also contain a long chain alcohol diluent or dispersant, carboxymethyl cellulose, or similar dispersing agents. Other commonly used surfactants such as Tweens or Spans or other similar emulsifying agents or bioavailability enhancers which are commonly used in the manufacture of pharmaceutically acceptable solid, liquid, or other dosage forms can also be used for the purpose of formulation.

The dosage required depends on the choice of the route of administration; the nature of the formulation; the nature of the subject's illness; the subject's size, weight, surface area, age, and sex; other drugs being administered; and the judgment of the attending physician. Suitable dosages may be in the range of 0.01-100.0 mg/kg. Wide variations in the needed dosage are to be expected in view of the variety of compositions available and the different efficiencies of various routes of administration. Variations in these dosage levels can be adjusted using standard empirical routines for optimization as is well understood in the art. Encapsulation of the composition in a suitable delivery vehicle (e.g., polymeric microparticles or implantable devices) may increase the efficiency of delivery, particularly for oral delivery.

The above-described pharmaceutical composition can be formulated into dosage forms for different administration routes utilizing conventional methods. For example, it can be formulated in a capsule, a gel seal, or a tablet for oral administration. Capsules can contain any standard pharmaceutically acceptable materials such as gelatin or cellulose. Tablets can be formulated in accordance with conventional procedures by compressing mixtures of the composition with a solid carrier and a lubricant. Examples of solid carriers include starch and sugar bentonite. The composition can also be administered in a form of a hard shell tablet or a capsule containing a binder, e.g., lactose or mannitol, a conventional filler, and a tableting agent. The pharmaceutical composition can be administered via the parenteral route. Examples of parenteral dosage forms include aqueous solutions, isotonic saline or 5% glucose of the active agent, or other well-known pharmaceutically acceptable excipient. Cyclodextrins, or other solubilizing agents well known to those familiar with the art, can be utilized as pharmaceutical excipients for delivery of the therapeutic agent.

The efficacy of the above-described pharmaceutical composition can be evaluated both in vitro and in vivo. Briefly, the pharmaceutical composition can be tested for its ability to inhibit PGR3/ZADH2 activity or expression in vitro. For in vivo studies, the pharmaceutical composition can be injected into an animal (e.g., a mouse model) and its therapeutic effects are then accessed. Based on the results, an appropriate dosage range and administration route can be determined.

The invention further features a method of identifying a compound for inhibiting PGR3/ZADH2 activity or expression. The compound can be designed, e.g., using computer modeling programs, according to the three-dimensional conformation of the polypeptide, and synthesized using methods known in the art. It can also be identified by library screening, or obtained using any of the numerous approaches in combinatorial library methods known in the art. Suitable libraries include: peptide libraries, peptoid libraries (libraries of molecules having the functionalities of peptides, but with a novel, non-peptide backbone that is resistant to enzymatic degradation), spatially addressable parallel solid phase or solution phase libraries, synthetic libraries obtained by deconvolution or affinity chromatography selection, the "one-bead one-compound" libraries, and antibody libraries. See, e.g., Zuckermann et al. (1994) J. Med. Chem. 37, 2678-85; Lam (1997) Anticancer Drug Des. 12, 145; Lam et al. (1991) Nature 354, 82; Houghten et al. (1991) Nature 354, 84; and Songyang et al. (1993) Cell 72, 767. Examples of methods for the synthesis of molecular libraries can be found in the art, for example, in: DeWitt et al. (1993) Proc. Natl. Acad. Sci. USA 90, 6909; Erb et al. (1994) Proc. Natl. Acad. Sci. USA 91, 11422; Zuckermann et al. (1994) J. Med. Chem. 37, 2678; Cho et al. (1993) Science 261, 1303; Carrell et al. (1994) Angew. Chem. Int. Ed. Engl. 33, 2059; Carell et al. (1994) Angew. Chem. Int. Ed. Engl. 33, 2061; and Gallop et al. (1994) J. Med. Chem. 37,1233. Libraries of compounds may be presented in solution (e.g., Houghten (1992) Biotechniques 13, 412-421), or on beads (Lam (1991) Nature 354, 82-84), chips (Fodor (1993) Nature 364, 555-556), bacteria (U.S. Patent No. 5,223,409), spores (U.S. Patent No. 5,223,409), plasmids (Cull et al. (1992) Proc. Natl. Acad. Sci. USA 89, 1865-1869), or phages (Scott and Smith (1990) Science 249, 386-390; Devlin (1990) Science 249, 404-406; Cwirla et al. (1990) Proc. Natl. Acad. Sci. USA 87, 6378-6382; Felici (1991) J. Mol. Biol. 222, 301-310; and U.S. Patent No. 5,223,409).

The specific examples below are to be construed as merely illustrative, and not limitative of the remainder of the disclosure in any way whatsoever. Without further elaboration, it is believed that one skilled in the art can, based on the description herein, utilize the present invention to its fullest extent. All publications cited herein are hereby incorporated by reference in their entirety.

### Example 1. Identification of a novel 15-keto prostaglandin-Δ¹³-reductase

The full-length cDNA sequences corresponding to the coding regions in the genes of GenBank Accession No. NM146090 and BC033780 were referred to as mouse PGR3/ZADH2 and human PGR3/ZADH2, respectively. The mouse PGR3/ZADH2 cDNA was prepared using mRNA extracted from 3T3-L1 adipocytes by PCR and ligated into a pGEM-T easy vector (Promega) by T4 DNA ligase (Promega). The sequences of the forward/reverse primers for obtaining mouse and human PGR3/ZADH2 listed below:
Mouse:
   Forward 5'-ATTGGATCCCAAATGCTGAGGCTGGCGGCC-3' (SEQ ID NO:5)
   Reverse 5'-ACGATGAATTCACAGCTTACTGCTGACAG-3' (SEQ ID NO:6) Human:
      Forward 5'-CGCGGATCCTTATGCTGCGGCTGGTGCCCAC-3'(SEQ ID NO:7)
      Reverse 5'- CCGGAATTCTTACAGCTTACTGTTGACAGAGTG-3'(SEQ ID NO:8)).

The deduced amino acid sequence of human PGR3/ZADH2 (SEQ ID NO:1) and mouse SEQ ID NO:3 are shown above. The mouse PGR3/ZADH2 was found to be homologous to human ZADH1 (GenBank accession no.: NM152444) but without significant similarity.

PGR3/ZADH2 expression increased during adipogenesis in 3T3-L1 cells. The maximal expression was observed at day 3 after induction of adipogenesis. At this time point, lipid droplets were observed to accumulate extensively in the adipocytes. The maximal PGR3/ZADH2 protein level was detected in fully differentiated adipocytes. It was also found that PPAR-gamma was induced markedly at an earlier stage of adipogenesis.

The tissue distribution of PGR3/ZADH2 was also determined. It was highly expressed in adipose tissue. The amount of PGR3/ZADH2 mRNA in omental fat was significantly higher in both homozygous and heterozygous db/db mice than in wild type mice.

Both human and mouse PGR3/ZADH2 proteins were recombinantly expressed in E.coli as GST fusion proteins following standard procedures. The recombinant PGR3/ZADH2 proteins thus obtained were used to determine substrate specificity and enzymatic kinetics.

Enzymatic activity was determined as follows. 10 µg of the recombinant mouse or human PGR3/ZADH2 protein was incubated at 37°C in a reaction buffer containing 0.1M Tris-HCl (pH7.4), 0.5 mM NADPH, and 0.57 mM 15-keto PGE₂. The reaction was conducted at 37°C in the dark for 10 minutes and terminated by adding 700 µl of a buffer which contained 50 mM potassium hydrogen phthalate, pH 3.0, and 1% Tween 20. 200 µl of a color development reagent, which contained 790 µM indonitrotetrazolium chloride, 60 µM phenazene methosulfate, and 1% Tween 20, was added to oxidize any unreacted NADPH. Absorbance at 490 nm was measured by an ELISA plate reader. A standard curve was generated using reaction buffers containing serially diluted amounts of NADPH.

Substrate specificity of PGR3/ZADH2 was determined using the just-described procedure, except that 15-keto PGE₂ was replaced with each of six prostaglandin substrates, each of three downstream metabolites, or leukotriene B4. 15-keto PGE₁, 15-keto PGF_{1α}, and 15-keto PGF_{2α} reacted specifically with PGR-3. By contrast, no specific activity was detected from 6-keto PGF1_{α}, PGF_{2β}, 11b-PGF_{2α}, 13,14-dihydro-15-keto PGF_{2α}, 13,14-dihydro-15-keto PGD₂, 13,14-dihydro-15-keto PGE₂, or leukotriene B4.

Also investigated was the effect of PGR-3/ZADH2 expression on modulating PPAR-gamma transcription in human Hep3B cells, which expressed endogenous human PPAR-alpha and -gamma. Over-expression of PGR3/ZADH2 in Hep3B cells was found to suppress PPAR-mediated transcriptional activation. The transcriptional activation was also suppressed even after Hep3B cells were stimulated by a PPAR-gamma agonist, i.e., BRL49653. Similar results were obtained from 3T3-L1 cells.

### Example 2. Prostaglandin

The effect of prostaglandin on PPAR-gamma activity in adipocytes was investigated. After treatment with a medium that induces cell differentiation, 3T3-L1 cells were treated from day 2 to 4 during adipogenesis with 14 µM 15-keto PGE₂, 13,14-dihydro-15-keto PGE₂, 15-keto PGF_{2α}, 13,14-dihydro-15-keto PGF_{2α}, or 4.5 µM of BRL49653, a PPAR-gamma agonist. See Forman et al., Cell (1995) 83:803-812. At day 6, aggregates of lipid droplets were stained with oil-red O for observation. 15-keto PGE₂ effectively enhanced adipogenesis at a level similar to BRL49653. After being induced to differentiate for two days, the 3T3-L1 cells were transfected with a reporter gene. Both 15-keto PGE₂ and 15-keto PGF_{2α} enhanced endogenous PPAR activity significantly. By contrast, the corresponding downstream metabolites, i.e., 13,14-dihydro-15-keto PGE₂ and 13,14-dihydro-15-keto PGF_{2α}, failed to increase PPAR activity.

A luciferase reporter gene was transfected to 3T3-L1 cells together with the ligand-binding domain of PPAR-alpha, PPAR-gamma or PPAR-delta fused to a yeast GAL4 DNA-binding domain. 15-keto PGE₂ and 15-keto PGF_{2α} activated PPAR-gamma and, to a lesser degree, PPAR-alpha.

Also examined was the ability of 15-keto PGE₂ to induce protein expression of adipogenesis-specific, PPAR-gamma target genes, i.e., IRS-1 and -2. Substantial amounts of PPAR-gamma1 and PPAR-gamma2 protein were detected in 3T3-L1 cells when they were treated with insulin and dexamethasone, but not methylisobutylxanthine (MIX) alone. Addition of 15-keto PGE₂ and MIX with insulin and dexamethasone significantly enhanced PPAR-gamma1 and PPAR-gamma2 expression. 15-keto PGE₂ and BRL49653 strongly induced expression of aP2, an adipocyte-specific marker, even in the absence of MIX. In the presence of insulin and dexamethasone, BRL49653 treatment dramatically increased IRS-2 expression. 15-keto PGE₂ enhanced the expression to a level similar to MIX. Either insulin/dexamethasone or MIX induced IRS-1 expression. PPAR-gamma ligands including 15-keto PGE₂ and BRL49653 did not increase the amount of IRS-1 protein.

### Example 3. PGR3/ZADH2 small molecule inhibitors

Recombinant human PGR3/ZADH2 protein was expressed and its enzymatic activities examined. Similar to mouse PGR3/ZADH2, recombinant human PGR3/ZADH2 had 15-keto-prostaglandin-Δ¹³-reductase activity and catalyzed conversion of 15-keto prostaglandin into 13,14-dihydro-15-keto prostaglandins.

Compounds 1-49 were tested for their inhibitory effects on PGR3/ZADH2 activity. These compounds are either commercially available (e.g., from Sigma-Aldrich, St. Louis, MO) or can be prepared from methods known in the art. The inhibition assay was performed following the procedure described above. Different concentrations of PGR3/ZADH2 inhibitors were added to the reaction mixture and incubated for 2 hours at 37°C.

More specifically, 50 µM of compounds 8, 12, 13, and 27, and 25 µM of tri-fluorobenzyl-2-hydroxycinnamate (compound 22) were added to the PGR3/ZADH2 enzyme reaction system and the ability of enzyme to reduce 15-keto-PGE₂ were measured. The results were summarized in Tables 1 and 2 below.

**Table 1**

| % Activity | No Inhibitor | Compound 12 | Compound 13 | Compound 8 | Compound 27 |
|---|---|---|---|---|---|
| Human PGR3/ZADH2 | 100 | 27.5 | 51.1 | 25.6 | 91.1 |
| Mouse PGR3/ZADH2 | 100 | 6.4 | 37.1 | 11.4 | 87.4 |

**Table 2**

| % Activity | No Inhibitor | trifluorobenzyl-2-hydroxycinnamate |
|---|---|---|
| Human PGR3/ZADH2 | 100 | 29.6 |
| Mouse PGR3/ZADH2 | 100 | 27.4 |

As shown in Tables 1 and 2, 15-keto prostaglandin-Δ¹³-reductase activity of PGR3/ZADH2 was inhibited by the above five compounds. Further, it was found that all of compounds 1-7, 9-11, 14-26, and 28-49 at a concentration of 50 µM, inhibited the activity by more than 20%.

### Example 4. Effects of PGR3/ZADH2 inhibitors on insulin sensitivity

The effect of two of the above-described PGR3/ZADH2 inhibitors, i.e., compounds 12 and 28, on insulin sensitivity was examined as follows. 3T3-L1 cells were induced to differentiate in the same manner as described above. A glucose transport assay was performed by measuring uptake of 2-deoxy-D-[³H]glucose according to the method described in Fingar et al. (Endocrinology 134:728-735). 1.67 µM of the inhibitor or 45 nM of a PPAR-gamma agonist, i.e., AVANDIA, was added to the cells.

10 µM cytochalasin-B was used to measure background glucose uptake levels. 100 nM of insulin was used to stimulate glucose uptake. Insulin treatment alone in differentiated 3T3-L1 cells increased glucose uptake by more than 10 folds. In the presence of either of those two PGR3/ZADH2 inhibitors, glucose uptake was increased by 30 folds. It was found that the ability of these inhibitors to enhance glucose uptake was comparable to that of AVANDIA.

The effect of PGR3/ZADH2 inhibitors on reducing blood glucose in db/db mice was examined. Female db/db mice (10-11 week-old C57BLKS/J-m+/+*Lep*^{db}, The Jackson Laboratory) were housed 4/cage. Ten mice were injected intraperitoneally twice daily for 7 days with 5 mg/kg of trifluorobenzyl 2-hydroxycinnamate (compound 22), a PGR3 inhibitor (mice ^{#}1-^{#}6), or a placebo (mice ^{#}7-^{#}10, final concentration against body fluid: 0.2% DMSO/0.7% ethanol in PBS). Blood samples from the retro-orbital sinus of the mice were obtained prior to the first injection and 18 hours after the last injection. The glucose levels in the blood samples were measured by an electrode-type blood glucose meter (HORIBA, AntSense II, Japan). The results were summarized in Table 3 below. The last column shows % inhibition, which represents (B.G.._{before} - B.G.._{after})/B.G.._{before}X 100%. The results suggest that the PGR-3 inhibitor significantly reduced blood glucose levels in mice.

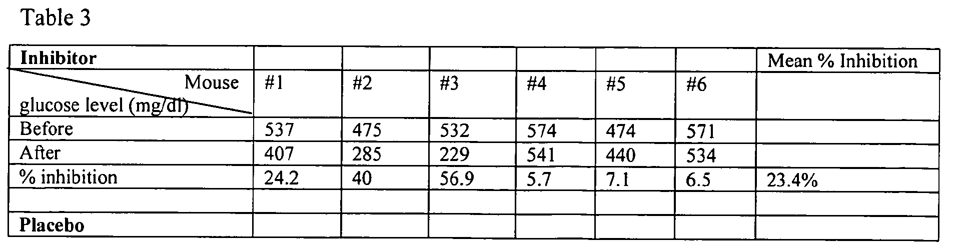

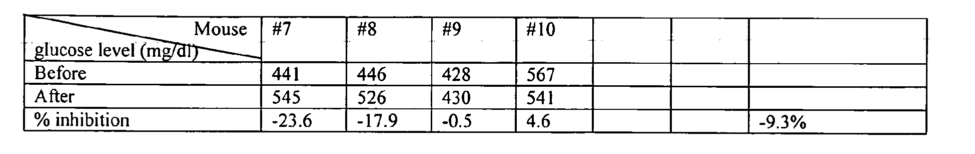

### Example 5. Interference RNA

An RNA interference (RNAi) approach was used to silence PGR3/ZADH2 expression in 3T3-L1 pre-adipocytes. To generate vectors that encode interference RNAs (iRNAs) targeting PGR3/ZADH2, oligonucleotides containing 5'-GAT CCG TGC CAC GTT ATC GGA ACC TTC AAG AGA GGT TCC GAT AAC GTG GCA CTT TTT TGG AAA-3' (SEQ ID NO:9; forward primer) and AGC TTT TCC AAA AAA GTG CCA CGT TAT CGG AAC CTC TCT TGA AGG TTC CGA TAA CGT GGC ACG-3'(SEQ ID NO: 10; reverse primer) were synthesized using a standard method. They were then annealed and incorporated into a linealized siRNA expression vector, pSilencerTM neo (catalog# 5764, Ambion). This siRNA expression vector was subsequently introduced into 3T3-L1 pre-adipocytes by electroporation. Stably-transfected clones were isolated by G418 selection. Levels of PGR3/ZADH2 mRNA in these claims were then examined by standard RT-PCR. It was found that that PGR3/ZADH2 mRNA transcription levels were repressed.

A PPRE-reporter based assay was conducted using the above-described clones according to the method described in Forman et al., Cell (1995) 83:803-812. It was found that the transcriptional activity of PPAR-gamma was increased in those clones. These results indicate that one can modulate PPAR-gamma activity via silencing PGR3/ZADH2 expression by RNA interference and thereby treat PPAR-related diseases.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions. Thus, other embodiments are also within the claims.

## Claims

1. An isolated polypeptide comprising a sequence of SEQ ID NO: 1 or SEQ ID NO:3 or a functional equivalent thereof, wherein the polypeptide reduces 15-keto prostaglandin.

2. The isolated polypeptide of claim 1, comprising a sequence at least 90% identical to SEQ ID NO: 1 or SEQ ID NO:3.

3. The isolated polypeptide of claim 2, comprising a sequence at least 80% identical to SEQ ID NO: 1 or SEQ ID NO:3.

4. The isolated polypeptide of claim 1, wherein the polypeptide is SEQ ID NO: 1 or 3.

5. The isolated polypeptide of claim 4, wherein the 15-keto prostaglandin is 15-keto PGE₂, 15-keto PGE₁, 15-keto PGF_{2α}, or 15-keto PGF_{1α}.

6. The isolated polypeptide of claim 5, wherein the 15-keto prostaglandin is 15-keto PGE₂.

7. An antibody, wherein the antibody binds specifically to a polypeptide of claim 1.

8. The antibody of claim 7, wherein the antibody is a monoclonal antibody.

9. The antibody of claim 7, wherein the polypeptide is SEQ ID NO: 1.

10. A method of treating a peroxisome proliferator-activated receptor-related disease, comprising administering to a subject in need thereof an effective amount of a modulator of PGR3/ZADH2.

11. The method of claim 10, wherein the modulator is 15-keto prostaglandin.

12. The method of claim 11, wherein the 15-keto prostaglandin is 15-keto PGE₂, 15-keto PGE1, 15-keto PGF2α, or 15-keto PGF1α.

13. The method of claim 12, wherein the 15-keto prostaglandin is 15-keto PGE₂.

14. The method of claim 10, wherein the modulator is a PGR3/ZADH2 inhibitor.

15. The method of claim 14, wherein the inhibitor is a dsRNA of claim 28.

16. The method of claim 14, wherein the inhibitor is tetrahydroxyflavone, trihydroxyflavone, 2-hydroxy-trimethoxychalcone, or 2-hydroxy-benzyl cinnamate, or a derivative thereof.

17. The method of claim 14, wherein the inhibitor is one of compounds 1-49.

18. The method of claim 14, wherein the inhibitor is an antibody.

19. The method of claim 10, wherein the PPAR related disease is type II diabetes, obesity, dyslipidemia, coronary heart disease, inflammatory disease, or cancer.

20. The method of claim 19, wherein the PPAR related disease is type II diabetes, obesity, dyslipidemia, or coronary heart disease.

21. The method of claim 20, wherein the PPAR related disease is type II diabetes.

22. A method of lowering blood glucose levels in a subject, comprising administering to the subject an effective amount of an inhibitor of PGR-3/ZADH2.

23. The method of claim 22, wherein the inhibitor is tetrahydroxyflavone, trihydroxyflavone, 2-hydroxy-trimethoxychalcone, or 2-hydroxy-benzyl cinnamate.

24. The method of claim 22, wherein the inhibitor is a dsRNA of claim 28 or a compound of compounds 1-49.

25. A method of identifying a compound for inhibiting activity of PGR3/ZADH2, comprising providing a system containing PGR3/ZADH2, contacting a compound with the system, determining the activity of PGR3/ZADH2, and comparing the activity with that obtained in the same manner except that the compound is absent.

26. The method of claim 25, wherein the system is a cell containing a gene that expresses PGR3/ZADH2 and the activity is determined by measuring expression activity of the gene.

27. The method of claim 25, wherein the system is a cell-free solution containing PGR3/ZADH2 and the activity is determined by measuring enzymatic activity of PGR3/ZADH2.

28. A double-stranded ribonucleic acid (dsRNA) for inhibiting expression of PGR3/ZADH2, comprising a first strand of polyribonucleotide and a second strand of polyribonucleotide, wherein the first strand contains a sequence that is identical to 19 to 49 consecutive nucleotides of a nucleic acid that encodes PGR3/ZADH2, and the second strand is complementary to the first strand.

29. The double-stranded ribonucleic acid of claim 28, wherein the first strand contains SEQ ID NO: 9 or 10, or a fragment thereof.

30. A method of treating a PPAR-related disease, comprising administering to a subject in need thereof an effective amount of a compound of formula (I): 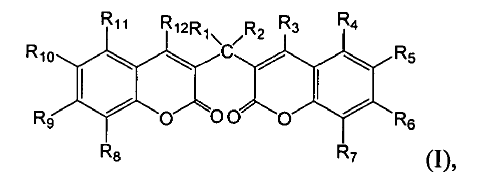 wherein
each of R₁ and R₂, independently, is H, halo, ORₐ, C₁-C₁₀ alkyl, C₃-C₂₀ cycloalkyl, C₃-C₂₀ heterocycloalkyl, aryl, or heteroaryl; and
each of R₃, R₄, R₅, R₆, R₇, R₈, R₉, R₁₀, R₁₁, and R₁₂, independently, is H, halo, OR_{b}, C₁-C₁₀ alkyl, C₃-C₂₀ cycloalkyl, C₃-C₂₀ heterocycloalkyl, aryl, or heteroaryl; or R₄ and R₅, taken together, are C₃-C₂₀ cycloalkyl, C₃-C₂₀ heterocycloalkyl, aryl, or heteroaryl; or R₁₀ and R₁₁, taken together, are C₃-C₂₀ cycloalkyl, C₃-C₂₀ heterocycloalkyl, aryl, or heteroaryl;
in which each of Rₐ and R_{b}, independently, is H, C₁-C₁₀ alkyl, C₃-C₂₀ cycloalkyl, C₃-C₂₀ heterocycloalkyl, aryl, or heteroaryl.

31. The method of claim 30, wherein each of R₁ and R₂, independently, is H, phenyl fused with cyclopentyl, or phenyl substituted with heteroaryl.

32. The method of claim 30, wherein each of R₃ and R₁₂ is OH.

33. A method of treating a PPAR-related disease, comprising administering to a subject in need thereof an effective amount of a compound of formula (II): 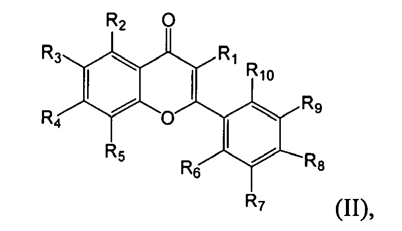 wherein
each of R₁, R₂, R₃, R₄, R₅, R₆, R₇, R₈, R₉, and R₁₀, independently, is H, OR, C₁-C₁₀ alkyl, C₃-C₂₀ cycloalkyl, C₃-C₂₀ heterocycloalkyl, aryl, or heteroaryl;
in which R is H, C₁-C₁₀ alkyl, C₃-C₂₀ cycloalkyl, C₃-C₂₀ heterocycloalkyl, aryl, or heteroaryl.

34. The method of claim 33, wherein each of R₁, R₂, R₃, R₄, R₅, R₆, R₇, R₈, R₉, and R₁₀, independently, is H or OH.

35. A method of treating a PPAR-related disease, comprising administering to a subject in need thereof an effective amount of a compound of formula (III): 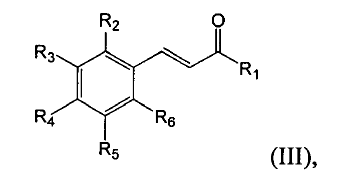 wherein
R₁ is C₁-C₁₀ alkyl, C₃-C₂₀ cycloalkyl, C₃-C₂₀ heterocycloalkyl, aryl, heteroaryl, ORₐ, NRₐR_{b}, or SRₐ; and
each of R₂, R₃, R₄, R₅, and R₆, independently, is H, halo, OR_{c}, NR_{c}R_{d}, C₁-C₁₀ alkyl, C₃-C₂₀ cycloalkyl, C₃-C₂₀ heterocycloalkyl, aryl, or heteroaryl;
in which each of Rₐ, R_{b}, R_{c}, and R_{d}, independently, is H, C₁-C₁₀ alkyl, C₃-C₂₀ cycloalkyl, C₃-C₂₀ heterocycloalkyl, aryl, or heteroaryl.

36. The method of claim 35, wherein R₁ is phenyl substituted with halo, OR, NO₂, or C₁-C₁₀ alkyl, in which R is H or C₁-C₁₀ alkyl.

37. The method of claim 35, wherein R₁ is ORₐ, NRₐR_{b}, or SRₐ, in which Rₐ is C₁-C₁₀ alkyl substituted with phenyl, phenyl being optionally substituted with CF₃, F, or OH; and R_{b} is H.

38. The method of claim 35, wherein R₁ is heteroaryl, or C₁-C₁₀ alkyl substituted with C(O)R, in which R is H or C₁-C₁₀ alkyl.

39. A method of treating a PPAR-related disease, comprising administering to a subject in need thereof an effective amount of a compound selected from the group consisting of


### Annex to the application documents - subsequently filed sequences listing

SEQUENCE LISTING

<110> Lin, Rong-Hwa
      Chuang, Lee-Ming

<120> MODULATION OF PEROXISOME
  PROLIFERATION-ACTIVATED RECEPTORS

<130> 13062-015001

<150> US 60/707,897
<151> 2005-08-12

<160> 10

<170> FastSEQ for Windows Version 4.0

<210> 1
<211> 377
<212> PRT
<213> Homo sapiens

<400> 1
Met Leu Arg Leu Val Pro Thr Gly Ala Arg Ala Ile Val Asp Met Ser
 1               5                  10                  15
Tyr Ala Arg His Phe Leu Asp Phe Gln Gly Ser Ala Ile Pro Gln Ala
            20                  25                  30
Met Gln Lys Leu Val Val Thr Arg Leu Ser Pro Asn Phe Arg Glu Ala
        35                  40                  45
Val Thr Leu Ser Arg Asp Cys Pro Val Pro Leu Pro Gly Asp Gly Asp
    50                  55                  60
Leu Leu Val Arg Asn Arg Phe Val Gly Val Asn Ala Ser Asp Ile Asn
65                  70                  75                  80
Tyr Ser Ala Gly Arg Tyr Asp Pro Ser Val Lys Pro Pro Phe Asp Ile
                85                  90                  95
Gly Phe Glu Gly Ile Gly Glu Val Val Ala Leu Gly Leu Ser Ala Ser
            100                 105                 110
Ala Arg Tyr Thr Val Gly Gln Ala Val Ala Tyr Met Ala Pro Gly Ser
        115                 120                 125
Phe Ala Glu Tyr Thr Val Val Pro Ala Ser Ile Ala Thr Pro Val Pro
    130                 135                 140
Ser Val Lys Pro Glu Tyr Leu Thr Leu Leu Val Ser Gly Thr Thr Ala
145                 150                 155                 160
Tyr Ile Ser Leu Lys Glu Leu Gly Gly Leu Ser Glu Gly Lys Lys Val
                165                 170                 175
Leu Val Thr Ala Ala Ala Gly Gly Thr Gly Gln Phe Ala Met Gln Leu
            180                 185                 190
Ser Lys Lys Ala Lys Cys His Val Ile Gly Thr Cys Ser Ser Asp Glu
        195                 200                 205
Lys Ser Ala Phe Leu Lys Ser Leu Gly Cys Asp Arg Pro Ile Asn Tyr
    210                 215                 220
Lys Thr Glu Pro Val Gly Thr Val Leu Lys Gln Glu Tyr Pro Glu Gly
225                 230                 235                 240
Val Asp Val Val Tyr Glu Ser Val Gly Gly Ala Met Phe Asp Leu Ala
                245                 250                 255

Val Asp Ala Leu Ala Thr Lys Gly Arg Leu Ile Val Ile Gly Phe Ile
            260                 265                 270
Ser Gly Tyr Gln Thr Pro Thr Gly Leu Ser Pro Val Lys Ala Gly Thr
        275                 280                 285
Leu Pro Ala Lys Leu Leu Lys Lys Ser Ala Ser Val Gln Gly Phe Phe
    290                 295                 300
Leu Asn His Tyr Leu Ser Lys Tyr Gln Ala Ala Met Ser His Leu Leu
305                 310                 315                 320
Glu Met Cys Val Ser Gly Asp Leu Val Cys Glu Val Asp Leu Gly Asp
                325                 330                 335
Leu Ser Pro Glu Gly Arg Phe Thr Gly Leu Glu Ser Ile Phe Arg Ala
            340                 345                 350
Val Asn Tyr Met Tyr Met Gly Lys Asn Thr Gly Lys Ile Val Val Glu
        355                 360                 365
Leu Pro His Ser Val Asn Ser Lys Leu
    370                 375

<210> 2
<211> 1134
<212> DNA
<213> Homo sapiens

<220>
<221> CDS
<222> (1)...(1131)

<400> 2
atg ctg cgg ctg gtg ccc acc ggg gcc cgg gcc atc gtg gac atg tcg    48
Met Leu Arg Leu Val Pro Thr Gly Ala Arg Ala Ile Val Asp Met Ser
 1               5                  10                  15

tac gcc cgc cac ttc ctg gac ttc cag ggc tcc gcc att ccc caa gcc    96
Tyr Ala Arg His Phe Leu Asp Phe Gln Gly Ser Ala Ile Pro Gln Ala
            20                  25                  30

atg cag aag ctg gtg gtg acc cgg ctg agc ccc aac ttc cgc gag gcc    144
Met Gln Lys Leu Val Val Thr Arg Leu Ser Pro Asn Phe Arg Glu Ala
        35                  40                  45

gtc acc ctg agc cgg gac tgc ccg gtg ccg ctc ccc ggg gac gga gac    192
Val Thr Leu Ser Arg Asp Cys Pro Val Pro Leu Pro Gly Asp Gly Asp
    50                  55                  60

ctc ctc gtc cgg aac cga ttt gtt ggt gtt aac gca tct gac atc aac    240
Leu Leu Val Arg Asn Arg Phe Val Gly Val Asn Ala Ser Asp Ile Asn
65                  70                  75                  80

tat tca gca ggc cgc tat gac ccc tca gtt aag cct ccc ttt gac ata    288
Tyr Ser Ala Gly Arg Tyr Asp Pro Ser Val Lys Pro Pro Phe Asp Ile
                85                  90                  95

ggt ttc gaa ggc att ggg gag gtg gtg gcc cta ggc ctc tct gct agt    336
Gly Phe Glu Gly Ile Gly Glu Val Val Ala Leu Gly Leu Ser Ala Ser
            100                 105                 110

gcc aga tac aca gtt ggc caa gct gtg gct tac atg gca cct ggt tct    384

Ala Arg Tyr Thr Val Gly Gln Ala Val Ala Tyr Met Ala Pro Gly Ser
        115                 120                 125

ttt gct gag tac aca gtt gtg cct gcc agc att gca act cca gtg ccc 432
Phe Ala Glu Tyr Thr Val Val Pro Ala Ser Ile Ala Thr Pro Val Pro
    130                 135                 140

tca gtg aaa ccc gag tat ctt acc ctg ctg gta agt ggc acc acc gca 480
Ser Val Lys Pro Glu Tyr Leu Thr Leu Leu Val Ser Gly Thr Thr Ala
145                 150                 155                 160

tac atc agc ctg aaa gag ctc gga gga ctg tcg gaa ggg aaa aaa gtt 528
Tyr Ile Ser Leu Lys Glu Leu Gly Gly Leu Ser Glu Gly Lys Lys Val
                165                 170                 175

ttg gtg aca gca gca gct ggg gga acg ggc cag ttt gcc atg cag ctt 576
Leu Val Thr Ala Ala Ala Gly Gly Thr Gly Gln Phe Ala Met Gln Leu
            180                 185                 190

tca aag aag gca aag tgc cat gta att gga acc tgc tct tct gat gaa 624
Ser Lys Lys Ala Lys Cys His Val Ile Gly Thr Cys Ser Ser Asp Glu
        195                 200                 205

aag tct gct ttt ctg aaa tct ctt ggc tgt gat cgt cct atc aac tat 672
Lys Ser Ala Phe Leu Lys Ser Leu Gly Cys Asp Arg Pro Ile Asn Tyr
    210                 215                 220

aaa act gaa ccc gta ggt acc gtc ctt aag cag gag tac cct gaa ggt 720
Lys Thr Glu Pro Val Gly Thr Val Leu Lys Gln Glu Tyr Pro Glu Gly
225                 230                 235                 240

gtc gat gtg gtc tat gaa tct gtt ggg gga gcc atg ttt gac ttg gct 768
Val Asp Val Val Tyr Glu Ser Val Gly Gly Ala Met Phe Asp Leu Ala
                245                 250                 255

gta gac gcc ctg gct acg aaa ggg cgc ttg ata gta ata ggg ttt atc 816
Val Asp Ala Leu Ala Thr Lys Gly Arg Leu Ile Val Ile Gly Phe Ile
            260                 265                 270

tct ggc tac caa act cct act ggc ctt tcg cct gtg aaa gca gga aca 864
Ser Gly Tyr Gln Thr Pro Thr Gly Leu Ser Pro Val Lys Ala Gly Thr
        275                 280                 285

ttg cca gcc aaa ctg ctc aag aaa tct gcc agc gta cag ggc ttc ttc 912
Leu Pro Ala Lys Leu Leu Lys Lys Ser Ala Ser Val Gln Gly Phe Phe
    290                 295                 300

ctg aac cat tac ctt tct aag tat caa gca gcc atg agc cac ttg ctc 960
Leu Asn His Tyr Leu Ser Lys Tyr Gln Ala Ala Met Ser His Leu Leu
305                 310                 315                 320

gag atg tgt gtg agc gga gac ctg gtt tgt gag gtg gac ctt gga gat 1008
Glu Met Cys Val Ser Gly Asp Leu Val Cys Glu Val Asp Leu Gly Asp
                325                 330                 335

ctg tct cca gag ggc agg ttt act ggc ctg gag tcc ata ttc cgt gct 1056
Leu Ser Pro Glu Gly Arg Phe Thr Gly Leu Glu Ser Ile Phe Arg Ala

340                 345                 350

gtc aat tat atg tac atg gga aaa aac act gga aaa att gta gtt gaa     1104
Val Asn Tyr Met Tyr Met Gly Lys Asn Thr Gly Lys Ile Val Val Glu
        355                 360                 365

tta cct cac tct gtc aac agt aag ctg taa                             1134
Leu Pro His Ser Val Asn Ser Lys Leu
    370                 375

<210> 3
<211> 377
<212> PRT
<213> Mus musculus

<400> 3
Met Leu Arg Leu Ala Ala Ala Gly Ala Arg Ala Ile Val Asp Met Ser
1               5                   10                  15
Tyr Ala Arg His Phe Leu Asp Phe Gln Gly Ser Ala Ile Pro Arg Thr
            20                  25                  30
Met Gln Lys Leu Val Val Thr Arg Leu Ser Pro Asn Phe His Glu Ala
        35                  40                  45
Val Thr Leu Arg Arg Asp Cys Pro Val Pro Leu Pro Gly Asp Gly Asp
    50                  55                  60
Leu Leu Val Arg Asn Arg Phe Val Gly Ile Asn Ala Ser Asp Ile Asn
65                  70                  75                  80
Tyr Ser Ala Gly Arg Tyr Asp Pro Ser Leu Lys Pro Pro Phe Asp Ile
                85                  90                  95
Gly Phe Glu Gly Ile Gly Glu Val Val Ala Leu Gly Leu Ser Ala Ser
            100                 105                 110
Ala Arg Tyr Thr Val Gly Gln Ala Val Ala Tyr Met Ala Pro Gly Ser
        115                 120                 125
Phe Ala Glu Tyr Thr Val Val Pro Ala Ser Ile Ala Ile Pro Met Pro
    130                 135                 140
Ser Val Lys Pro Glu Tyr Leu Thr Met Leu Val Ser Gly Thr Thr Ala
145                 150                 155                 160
Tyr Leu Ser Leu Glu Glu Leu Gly Glu Leu Ser Glu Gly Lys Lys Val
                165                 170                 175
Leu Val Thr Ala Ala Ala Gly Gly Thr Gly Gln Phe Ala Val Gln Leu
            180                 185                 190
Ser Lys Ile Ala Lys Cys His Val Ile Gly Thr Cys Ser Ser Asp Glu
        195                 200                 205
Lys Ala Ala Phe Leu Lys Ser Ile Gly Cys Asp Arg Pro Ile Asn Tyr
    210                 215                 220
Arg Thr Glu Pro Val Glu Thr Val Leu Lys Gln Glu Tyr Pro Glu Gly
225                 230                 235                 240
Val Asp Val Val Tyr Glu Ser Val Gly Gly Ala Met Phe Asp Leu Ala
                245                 250                 255
Val Asp Ala Leu Ala Thr Lys Gly Arg Leu Ile Val Ile Gly Phe Ile
            260                 265                 270
Ser Gly Tyr Gln Ser Pro Thr Gly Leu Ser Pro Ile Lys Ala Gly Val
        275                 280                 285
Leu Pro Thr Lys Leu Leu Lys Lys Ser Ala Ser Leu Arg Gly Phe Phe
    290                 295                 300
Leu Asn His Tyr Phe Ser Lys Tyr Gln Ala Ala Met Glu Arg Leu Leu
305                 310                 315                 320

Glu Leu Tyr Ala Arg Gly Asp Leu Val Cys Glu Val Asp Leu Gly His
                325                 330                 335
Leu Ala Pro Asp Gly Arg Phe Ile Gly Leu Glu Ser Val Phe Gln Ala
            340                 345                 350
Val Asp Tyr Met Tyr Thr Gly Lys Asn Thr Gly Lys Leu Val Val Glu
        355                 360                 365
Leu Pro His Pro Val Ser Ser Lys Leu
    370                 375

<210> 4
<211> 1134
<212> DNA
<213> Mus musculus

<400> 4
atgctgaggc tggcggccgc cggggcccga gccatcgtgg acatgtcgta cgcccgtcac 60
ttcctggact tccagggctc cgccatcccc cgaaccatgc agaagctggt ggtgacccgg 120
ctgagcccta acttccacga ggccgtcacc ctgcgccggg actgcccagt gccgctcccc 180
ggggacggag acctcctcgt ccggaaccga ttcgttggta ttaacgcatc tgacatcaac 240
tattcggctg gccgctacga cccgtccctg aagccaccct ttgacatagg ttttgaaggg 300
attggtgagg tggtggcctt aggcctctct gctagtgcta ggtacacagt gggccaggct 360
gtggcttata tggctcctgg ttcctttgct gagtatacag tggtgcctgc tagcattgca 420
attcccatgc cttcagtgaa accagagtat ctcaccatgc tggttagtgg caccactgca 480
tacctcagcc tggaagagct tggggaactg tcagaaggga agaaagttct ggtcacagca 540
gccgctgggg gcacaggcca gtttgctgtg cagctttcca agatagccaa gtgccacgtt 600
atcggaacct gctcctcaga cgaaaaggca gcttttctga aatcaattgg gtgtgatcgg 660
cccatcaact acagaacaga gcctgtggag acggttctga agcaggagta ccctgaaggc 720
gttgacgtag tctatgagtc tgttggggga gccatgtttg acctggctgt ggatgccttg 780
gccaccaaag ggcgcttgat agtgattggg tttatctctg gctaccaaag ccctacagga 840
ctctcaccaa taaaagcggg agttttgcca accaagctcc tgaagaagtc ggccagcctc 900
aggggtttct ttctgaacca ctacttctcc aagtaccagg ctgccatgga acgtttgctg 960
gagctgtacg ctcgtgggga cctggtgtgt gaggtggacc tgggacacct ggctccggat 1020
ggaaggttca ttggcctgga gtccgtgttt caggctgtcg actatatgta cacggggaaa 1080
aatactggga agcttgttgt tgagttacca caccctgtca gcagtaagct gtga 1134

<210> 5
<211> 30
<212> DNA
<213> Artificial Sequence

<220>
<223> Primer

<400> 5
attggatccc aaatgctgag gctggcggcc 30

<210> 6
<211> 29
<212> DNA
<213> Artificial Sequence

<220>
<223> Primer

<400> 6
acgatgaatt cacagcttac tgctgacag 29

<210> 7
<211> 31
<212> DNA
<213> Artificial Sequence

<220>
<223> Primer

<400> 7
cgcggatcct tatgctgcgg ctggtgccca c                                    31

<210> 8
<211> 33
<212> DNA
<213> Artificial Sequence

<220>
<223> Primer

<400> 8
ccggaattct tacagcttac tgttgacaga gtg                                  33

<210> 9
<211> 63
<212> DNA
<213> Artificial Sequence

<220>
<223> Primer

<400> 9
   gatccgtgcc acgttatcgg aaccttcaag agaggttccg ataacgtggc acttttttgg 60
   aaa                                                                   63

<210> 10
<211> 63
<212> DNA
<213> Artificial Sequence

<220>
<223> Primer

<400> 10
agcttttcca aaaaagtgcc acgttatcgg aacctctctt gaaggttccg ataacgtggc 60
acg                                                                   63